(19) Europäisches Patentamt
European Patent Office
Office européen des brevets

(11) **EP 3 153 857 A1**

(12) **EUROPEAN PATENT APPLICATION**

(43) Date of publication:
**12.04.2017 Bulletin 2017/15**

(51) Int Cl.:
*G01N 33/52* (2006.01)    *G01N 21/78* (2006.01)
*G01N 33/543* (2006.01)    *G01N 31/22* (2006.01)

(21) Application number: **16182537.7**

(22) Date of filing: **03.08.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **08.10.2015 TW 104133131**

(71) Applicant: **Apex Biotechnology Corporation Hsinchu (TW)**

(72) Inventors:
• **Lu, Yi-Chen**
  **Hsinchu (TW)**
• **Kuan, Tang-Ching**
  **Hsinchu (TW)**

(74) Representative: **Becker Kurig Straus Patentanwälte**
  **Bavariastrasse 7**
  **80336 München (DE)**

(54) **BIOCHEMICAL TEST CHIP**

(57)    Provided is a biochemical test chip including an insulating substrate, an electrode unit, a first insulating septum, a reactive layer, a second insulating septum, and a color-changing layer. The electrode unit is located on the insulating substrate. The first insulating septum is located on the electrode unit and has an opening. The opening exposes a portion of the electrode unit. The reactive layer is located in the opening. The second insulating septum is located on the first insulating septum. The color-changing layer is located at least on a region outside the reactive layer. The color of the color-changing layer is changed with a change in an environmental factor.

FIG. 1

**Description**

BACKGROUND OF THE INVENTION

Field of the Invention

The invention relates to a biochemical test chip, and more particularly, to a biochemical test chip having a conjugated polymer.

Description of Related Art

A traditional biochemical sensor analyzes the biochemical concentration generated by a reaction in which a reactive layer containing a component such as an enzyme, a conductive medium, or a buffer solution is reacted with a sample via an optical or an electrochemical method. However, the above components of the reactive layer are very sensitive to environmental factors such as light, temperature, humidity, and oxygen content. For instance, if an enzyme is not in an optimal temperature environment, the enzyme loses the original activity thereof. Moreover, the conductive medium also generates structural change due to light irradiation. For instance, a ferrous compound becomes a ferric compound after UV irradiation. Moreover, since the above reactive layer is mostly an irreversible reaction due to a change generated by an environmental factor, the biochemical sensor loses the original measurement function.

To eliminate the influence of the environmental factor on the biochemical sensor, an aluminum film or an opaque container containing desiccant is often used to store the biochemical sensor. However, the packaging method can only achieve limited separation (such as light or humidity), and cannot effectively eliminate the influence of temperature on the biochemical sensor, and does not have the function of prompting the user that the biochemical sensor is damaged by temperature.

Although a plurality of reaction region biochemical sensors has been designed, whether the biochemical sensor is damaged can be analyzed via the measurement of concentration of two reaction regions or a chemical reaction agent can be mixed in the reactive layer, so as to measure whether the reactive layer agent is damaged. However, in both of the above two methods, the user needs to place a sample in the reaction region to learn whether the sensor is damaged, thus causing inconvenience to the user. Therefore, how to develop a biochemical test chip capable of sensing a change in an environmental factor and capable of prompting the user whether the biochemical test chip is damaged without having to place a sample in the reaction region is a very important topic in the future.

SUMMARY OF THE INVENTION

The invention provides a biochemical test chip capable of sensing a change in an environmental factor and prompting a user whether the biochemical test chip is damaged without having to place a sample in a reaction region.

The invention provides a biochemical test chip including an insulating substrate, an electrode unit, a first insulating septum, a reactive layer, a second insulating septum, and a color-changing layer. The electrode unit is located on the insulating substrate. The first insulating septum is located on the electrode unit and has an opening. The opening exposes a portion of the electrode unit. The reactive layer is located in the opening. The second insulating septum is located on the first insulating septum. The color-changing layer is located at least on a region outside the reactive layer. The color of the color-changing layer is changed with a change in an environmental factor.

In an embodiment of the invention, the color-changing layer is disposed on the top or the bottom of the insulating substrate, the top or the bottom of the first insulating septum, or the top or the bottom of the second insulating septum.

In an embodiment of the invention, the material of the color-changing layer includes a conjugated polymer.

In an embodiment of the invention, the conjugated polymer includes an aromatic hydrocarbon compound, a non-aromatic hydrocarbon compound, a trans-aromatic hydrocarbon compound, or a combination thereof.

In an embodiment of the invention, the aromatic hydrocarbon compound includes a polycyclic aromatic hydrocarbon compound, a phenyl compound, a crystal violet derivative, or a combination thereof.

In an embodiment of the invention, the polycyclic aromatic hydrocarbon compound includes a triarylmethane compound, a fluoran compound, a phenothiazine compound, a thiofluoran compound, a xanthene compound, a spiropyran compound, a chromenopyrazole compound, a methine compound, a rhodaminelactam compound, a quinazoline compound, a diazaxanthene compound, a bislactone compound, or a combination thereof.

In an embodiment of the invention, the phenyl compound includes a cresol red derivative, a thymol blue derivative, an aniline yellow derivative, a 2,4-dinitrophenol derivative, a bromophenol blue derivative, a methyl orange derivative, a bromcresol green derivative, a methyl red derivative, an eriochrome black T derivative, a bromcresol purple derivative, an alizarin derivative, an m-nitrophenol derivative, an o-cresolphthalein derivative, a phenolphthalein derivative, a thymolphthalein derivative, an alizarin yellow R derivative, an indigo carmine derivative, a malachite green derivative, a phenyl tribromomethyl sulfone derivative, a Victoria blue B derivative, a Victoria green G derivative, a phthalocyanine derivative, a phthaloyanine green G derivative, or a combination thereof.

In an embodiment of the invention, the color-changing layer further includes a monomer. The monomer includes an amorphous polymer, chlorostyrene, ethylene, propene, butene, isoamylene, vinyl acetate, vinyl propionate, vinyl benzoate, vinyl butyrate, methyl acrylate, ethyl acrylate, butyl acrylate, 2-ethylhexyl acrylate, phenyl acrylate, methyl methacrylate, ethyl methacrylate, butyl methacrylate, vinyl methyl ether, ethoxyethene, butyl vinyl ether, methyl vinyl ketone, acetylacetone, polyvinyl, polypropylene, a polyester resin, a polyurethane resin, an epoxy resin, a silicone resin, a modified resin, paraffins, or a combination thereof.

In an embodiment of the invention, the color-changing layer further includes a sensitizer. The sensitizer includes an unsaturated ketone, a 1,2-diketone derivative, a benzo derivative, a fluorene derivative, a naphthoquinone derivative, an anthraquinone derivative, a xanthenes derivative, a coumarin derivative, a gallocyanine derivative, a merocyanine-based derivative, a polymethine derivative, an acridine derivative, a pyridazine derivative, an oxazine derivative, an indoline derivative, an azulene derivative, a porphyrins derivative, a tetraphenyl porphyrin derivative, a triarylmethane derivative, a phthalocyanin derivative, an annulene derivative, a spiropyrans derivative, a spirooxazine derivative, an organic ruthenium complex, or a combination thereof.

In an embodiment of the invention, the unsaturated ketone includes a flavones derivative, dibenzalacetone, or a combination thereof, and the 1,2-diketone derivative includes a benzyl derivative, a camphorquinone derivative, or a combination thereof.

In an embodiment of the invention, the forming method of the color-changing layer includes a coating method, an inkjet printing method, a mesh printing method, or a distributed printing method.

In an embodiment of the invention, the environmental factor includes light, temperature, humidity, oxygen content, or a combination thereof.

In an embodiment of the invention, the color change of the color-changing layer displays a text, a number, a figure, a pattern, a symbol, or a combination thereof.

In an embodiment of the invention, the color-changing layer further includes an initiator, and the initiator includes organic peroxide.

In an embodiment of the invention, the organic peroxide includes isobutyl peroxide, α,α'-bis(neodecanoylperoxy)diisopropylbenzene, cumyl peroxyneodecanoate, bis-n-propyl peroxydicarbonate, bis-s-butyl peroxydicarbonate, 1,1,3,3-tetramethylbutyl neodecanoate, bis(4-t-butylcyclohexyl)peroxydicarbonate, 1-cyclohexyl-1-methyl ethyl peroxyneodecanoate, bis-2-ethoxy ethyl peroxydicarbonate, bis(ethylhexylperoxy)dicarbonate, t-hexyl neodecanoate, bismethoxy butyl peroxydicarbonate, bis(3-methyl-3-methoxybutylperoxy)dicarbonate, t-butyl peroxyneodecanoate, t-hexyl peroxypivalate, 3,5,5-trimethyl hexanoyl peroxide, octanoyl peroxide, lauroyl peroxide, stearoyl peroxide, 1,1,3,3-tetramethyl butyl peroxy-2-ethyl hexanoate, succinic peroxide, 2,5-dimethyl-2,5-bis(2-ethylhexanoyl)hexane, 1-cyclohexyl-1-methylethyl peroxy-2-ethylhexanoate, t-hexyl peroxy-2-ethyl hexanoate, 4-methylbenzoyl peroxide, t-butyl peroxy-2-ethylhexanoate, m-toluoyl benzoyl peroxide, benzoyl peroxide, t-butylperoxy isobutyrate, 1,1-bis(t-butylperoxy)-2-methyl-cyclohexane, 1,1-bis(t-butylperoxy)-3,3,5-trimethylcyclohexane, 1,1-bis(t-hexylperoxy)cyclohexane, 1,1-bis(t-butylperoxy)-3,3,5-trimethylcyclohexane, 1,1-bis(t-butylperoxy)cyclohexane, 2,2-bis(4,4-di-butylperoxycyclohexyl)propane, 1,1-bis(t-butylperoxy)cyclododecane, t-hexylperoxy isopropyl monocarbonate, t-butylperoxy maleic acid, t-butyl peroxy-3,5,5-trimethylhexanoate, t-butylperoxy laurate, 2,5-dimethyl-2,5-bis(m-toluoylperoxy)hexane, t-butylperoxy isopropyl monocarbonate, t-butylperoxy-2-ethylhexyl monocarbonate, t-hexyl peroxybenzoate, 2,5-dimethyl-2,5-bis(benzoylperoxy)hexane, t-butylperoxyacetate, 2,2-bis(t-butylperoxy)butane, t-butylperoxybenzoate, n-butyl-4,4-bis(t-butylperoxy)valerate, di-t-butylperoxyisophthalate, α,α'-bis(t-butylperoxy)diisopropylbenzene, dicumylperoxide, 2,5-dimethyl-2,5-di(t-butylperoxy)hexane, t-butylcumylperoxide, p-menthane hydroperoxide, 2,5-dimethyl-2,5-bis(t-butylperoxy)hexyne, diisopropylbenzene hydroperoxide, t-butyl trimethylsilyl peroxide, 1,1,3,3-tetramethyl butyl hydroperoxide, cumene hydroperoxide, t-hexyl hydroperoxide, t-butyl hydroperoxide, 2,3-dimethyl-2,3-diphenylbutane, or a combination thereof.

In an embodiment of the invention, the activity rate of change of the reactive layer is equal to the concentration rate of change of the color-changing layer.

In an embodiment of the invention, the concentration rate of change of the color-changing layer is defined by formula (1) and formula (2) as shown below:

$$A = -\frac{\ln\left(\dfrac{C_i}{C_0}\right)}{K} \tag{1}$$

$$K = e^{\left(a + \frac{b}{T}\right)} \tag{2},$$

wherein,

K is a reaction rate constant of the color-changing layer defined by formula (2);

T is a temperature in Kelvin;

a and b are experiment constants, wherein a is an intercept of the reaction rate constant K of the color-changing layer and the temperature T, and b is a slope of the reaction rate constant K of the color-changing layer and the temperature T;

$C_0$ is an original concentration of the color-changing layer;

$C_i$ is a concentration change to be shown by the color-changing layer, wherein the concentration change is defined as a concentration change when the reactive layer loses the original measurement function;

A is a stability duration of the reactive layer or the color-changing layer at the temperature T.

In an embodiment of the invention, the reaction rate constant of the color-changing layer at a specific temperature is adjusted by the concentration of a compound in the color-changing layer.

In an embodiment of the invention, the compound in the color-changing layer includes a conjugated polymer, a monomer, a sensitizer, an initiator, or a combination thereof.

Based on the above, in the invention, via the color-changing layer disposed at least on a region outside the reactive layer and having a conjugated polymer, the color thereof can be changed with a change in an environmental factor. Moreover, the color change of the color-changing layer can be displayed as various texts, numbers, figures, patterns, or symbols. Therefore, the user can learn whether the biochemical test chip is damaged without having to place a sample in the reaction region. As a result, not only is wear and tear of the sample reduced, the risk of detection error of the biochemical test chip can also be reduced.

In order to make the aforementioned features and advantages of the disclosure more comprehensible, embodiments accompanied with figures are described in detail below.

BRIEF DESCRIPTION OF THE DRAWINGS

The accompanying drawings are included to provide a further understanding of the invention, and are incorporated in and constitute a part of this specification. The drawings illustrate embodiments of the invention and, together with the description, serve to explain the principles of the invention.

FIG. 1 is an exploded view of a biochemical test chip according to an embodiment of the invention.
FIG. 2 is a graph of activity loss of the reactive layers of experimental examples 1 and 2.

DESCRIPTION OF THE EMBODIMENTS

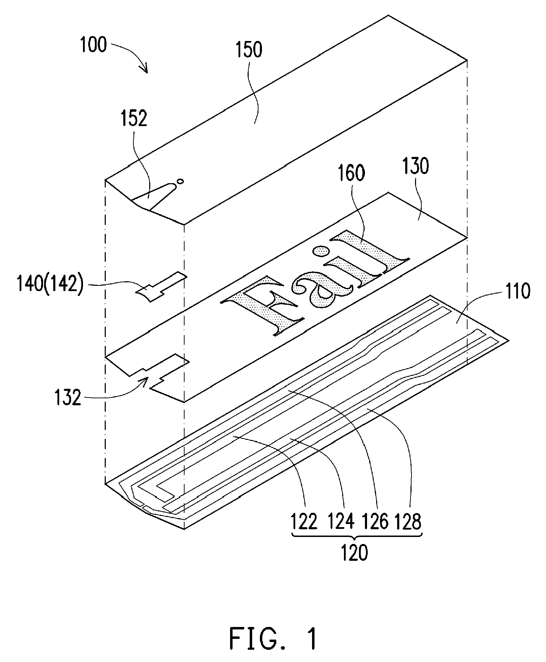

FIG. 1 is an exploded view of a biochemical test chip according to an embodiment of the invention.

Referring to FIG. 1, the invention provides a biochemical test chip 100 including an insulating substrate 110, an electrode unit 120, a first insulating septum 130, a reactive layer 140, a second insulating septum 150, and a color-changing layer 160. In the present embodiment, the biochemical test chip 100 is an electrochemical test piece used to receive the blood sample of a user for measuring the numeric value of, for instance, blood sugar, cholesterol, uric acid, lactic acid, or heme in blood. However, the invention is not limited thereto, and in other embodiments, the biochemical test chip 100 can also be used in any liquid-form sample, as long as the biochemical test chip 100 can generate an electrochemical reaction with the reactive layer 140 or has the capability of specificity identification of a biological material or signal.

The insulating substrate 110 is a substrate having a flat surface and having electrical insulation and heat resistance capability for 40 °C to 120 °C. In an embodiment, the material of the insulating substrate 110 can contain polyvinyl chloride (PVC), glass fiber (FR-4), polyester suphone, bakelite, polyethylene terephthalate (PET), polycarbonate (PC), polypropylene (PP), polyethylene (PE), polystyrene (PS), glass plate, ceramic, or any combination of the above materials. Of course, the material of the insulating substrate 110 is not limited thereto.

As shown in FIG. 1, the electrode unit 120 is located on the insulating substrate 110. The electrode unit 120 contains a working electrode 122 and a reference electrode 124 insulated from each other and identification electrodes 126 and 128. In the present embodiment, the identification electrodes 126 and 128 are disposed on the outside of the working electrode 122 and the reference electrode 124. However, the disposition of the electrode unit 120 may be different in response to various needs, and is not limited to the arrangement method between the electrodes, and the number of the electrodes is also not limited. The designer can change the number of the electrodes based on actual application, and the invention is not limited thereto.

In the present embodiment, the identification electrodes 126 and 128 can be turned on via a liquid-form sample entering from the opening 132 (such as a sampling port) in a subsequent process, so as to initiate the measuring step. The working electrode 122 and the reference electrode 124 are used to determine whether the liquid-form sample entering in a subsequent process reacts with the reactive layer 140 in an electrochemical reaction or generates a specificity identification biological signal. However, the invention is not limited thereto, and in another embodiment, the electrodes 126 and 128 can also be used to perform measurement of a disrupting object. For instance, when the electrodes 122 and 124 perform blood sugar measurement, the blood sugar value can be corrected via the measured value of the disrupting object. Moreover, in other embodiments, a first sample concentration detection can also be performed via the electrodes 126 and 128, and a second sample concentration detection can be performed via the electrodes 122 and 124. The material of the electrode unit 120 can be any conductive substance such as palladium adhesive, platinum adhesive, gold adhesive, titanium adhesive, carbon adhesive, silver adhesive, copper adhesive, gold and silver mixed adhesive, carbon and silver mixed adhesive, or any combination of the conductive materials. In an embodiment, the electrode unit 120 is formed by a conductive carbon powder layer. In another embodiment, the electrode unit 120 is formed by a metal layer. In another embodiment, the electrode unit 120 is composed of a conductive silver adhesive layer and a conductive carbon powder layer located thereon, and the impedance of the conductive carbon powder layer is generally far greater than that of a conductive silver adhesive layer or other metal adhesive layers.

The first insulating septum 130 is located on the electrode unit 120. The first insulating septum 130 has an opening 132, and the opening 132 exposes at least a portion of the working electrode 122 and the reference electrode 124. In the present embodiment, the opening 132 only needs to be able to expose a portion of the working electrode 122 and the reference electrode 124 needed for measurement, and the invention does not limit the area and the shape of the opening 132. In an embodiment, the material of the first insulating septum 130 can contain, but not limited to, PVC insulation tape, ethylene terephthalate insulating tape, thermal drying insulating paint, or UV-curing insulating paint.

The reactive layer 140 is located in the opening 132. The reactive layer 140 covers at least the working electrode 122 and the reference electrode 124 corresponding to the opening 132 to perform an electrochemical reaction or generate a specificity identification biological signal. Specifically, the reactive layer 140 contains at least oxidoreductase and an electron transfer regulator. The oxidoreductase has high specificity towards a target analyte (such as glucose, cholesterol, uric acid, lactic acid, or heme), only catalyzes the reaction configuration of the target analyte, and has polymer composite globular protein catalyzing the target analyte.

In another embodiment, the reactive layer 140 can also include a coenzyme participating in the reaction, wherein the coenzyme is a special subset prosthetic group, and the cofactor thereof is tightly adhered on the oxidoreductase, and is not consumed in the above reaction. Moreover, since the entire reaction is a coupling reaction, the consumption or the generation amount of the coenzyme can also be used to determine the catalytic activity of the oxidoreductase. In an embodiment, the coenzyme can be, for instance, flavin-adenine dinucleotide or nicotinamide adenine dinucleotide, but the invention is not limited thereto.

When the target analyte reacts with an oxidoreductase metabolite, the cofactor of the coenzyme is reduced, and the electron transfer regulator is used to return the coenzyme to the dehydrogenated state thereof. In other words, the coenzyme and the electron transfer regulator are reduced, and the reduced electrons are spread to the biochemical sensor electrode surface to form a potential difference. The analyte content of the analysis target is quantified by measuring the potential difference. In an embodiment, the electron transfer regulator can be, for instance, ferrocene and a derivative thereof, quinine and a derivative thereof, organic conductive salt or viologen, chloride hexamethyl tetraamine ruthenium (III), potassium ferricyanide, potassium ferrocyanide, or a combination thereof, but the invention is not limited thereto.

The second insulating septum 150 is located on the first insulating septum 130 and the reactive layer 140. Since the second insulating septum 150 completely covers the reactive layer 140, the top, the bottom, and the three sidewalls (except the sampling port) of the reactive layer 140 are surrounded by the second insulating septum 150, the insulating substrate 110, and the first insulating septum 130 to form a tubular space (not shown). When the liquid-form sample enters the tubular space, the adhesion of the liquid-form sample in the tubular space is greater than the cohesion of the liquid-form sample, such that the liquid-from sample continues to move forward. At this point, the liquid-form sample is in contact with the reactive layer 140 in the tubular space, such that the liquid-form sample is mixed with the oxidoreductase, the coenzyme, and the electron transfer regulator in the reactive layer 140 to form a reaction region 142 in the tubular space.

Moreover, to let the user see the state of injection of the liquid-form sample in the reaction region 142, in the present embodiment, the second insulating septum 150 has a transparent observing region 152. The transparent observing region 152 exposes at least a portion of the reaction region 142 to allow the observation of the state of injection of the liquid sample in the reaction region 142. In an embodiment, the second insulating septum 150 can also be, for instance, a transparent upper cover for observing the color change of the color-changing layer 160.

It should be mentioned that, in the present embodiment, the color-changing layer 160 is disposed at least on a region outside the reactive layer 140. The color of the color-changing layer 160 can be changed with a change in an environmental factor. For instance, as shown in FIG. 1, the color-changing layer 160 is disposed on the top of the first insulating septum 130. Since the material of the color-changing layer 160 includes a conjugated polymer, the conjugated polymer and the reactive layer 140 have the same rate of change, and the color-changing layer 160 and the reactive layer 140 are in the same environmental conditions (i.e., same light, temperature, humidity, and oxygen content). Therefore, when the conjugated polymer generates color change with a change in an environmental factor, the oxidoreductase, the coenzyme, or the electron transfer regulator in the reactive layer 140 also loses activity or generates structural change with time environmental factor. As a result, the user can learn whether the biochemical test chip 100 of the present embodiment is damaged via comparison colors pre-printed on a specimen jar, a specification, or a screen display of a measuring instrument. In an embodiment, the conjugated polymer can be, for instance, an aromatic hydrocarbon compound, a non-aromatic hydrocarbon compound, a trans-aromatic hydrocarbon compound, or a combination thereof. The aromatic hydrocarbon compound can be, for instance, a polycyclic aromatic hydrocarbon compound, a phenyl compound, a crystal violet derivative, or a combination thereof. The polycyclic aromatic hydrocarbon compound can be, for instance, a triarylmethane compound, a fluoran compound, a phenothiazine compound, a thiofluoran compound, a xanthene compound, a spiropyran compound, a chromenopyrazole compound, a methine compound, a rhodaminelactam compound, a quinazoline compound, a diazaxanthene compound, a bislactone compound, or a combination thereof. The phenyl compound can be, for instance, a cresol red derivative, a thymol blue derivative, an aniline yellow derivative, a 2,4-dinitrophenol derivative, a bromophenol blue derivative, a methyl orange derivative, a bromcresol green derivative, a methyl red derivative, an eriochrome black T derivative, a bromcresol purple derivative, an alizarin derivative, an m-nitrophenol derivative, an o-cresolphthalein derivative, a phenolphthalein derivative, a thymolphthalein derivative, an alizarin yellow R derivative, an indigo carmine derivative, a malachite green derivative, a phenyl tribromomethyl sulfone derivative, a Victoria blue B derivative, a Victoria green G derivative, a phthalocyanine derivative, a phthaloyanine green G derivative, or a combination thereof

In an embodiment, the forming method of the color-changing layer 160 can include a coating method, an inkjet printing method, a mesh printing method, or a distributed printing method. For instance, the conjugated polymer in liquid form can be coated on the top or the bottom of the insulating substrate 110, the top or the bottom of the first insulating septum 130, or the top or the bottom of the second insulating septum 150 via a nozzle. However, the invention is not limited thereto, as long as the conjugated polymer is coated on a region outside the reactive layer 140. Then, the conjugated polymer in liquid form is converted to a conjugated polymer in solid form. In an embodiment, the conversion method can be, for instance, natural drying or baking, and the invention is not limited thereto.

Moreover, since the color-changing layer 160 can be formed by the various methods above, when the color of the color-changing layer 160 is changed with a change in an environmental factor, the color change of the color-changing layer 160 can display a text, a number, a figure, a pattern, a symbol, or a combination thereof As a result, the user can learn whether the biochemical test chip is damaged without having to place a sample in the reaction region. As a result, not only is wear and tear of the sample reduced, the detection efficiency of the biochemical test chip can also be increased. In an embodiment, the environmental factor can be, for instance, light, temperature, humidity, oxygen content, or a combination thereof. However, the invention is not limited thereto, and any environmental factor causing the oxidoreductase, the coenzyme, or the electron transfer regulator in the reactive layer 140 to lose activity or generate structural change is included in the scope of the invention.

Furthermore, the invention does not limit the identification method of color change of the conjugated polymer in the color-changing layer 160. In an embodiment, at least two comparison colors (one of them is the color of the biochemical test chip without damage, and the other one is the color of a damaged biochemical test chip) can be printed on the biochemical test chip, the color-changing layer is located in the middle of the two comparison colors (not shown), and the user can identify whether the biochemical test chip is damaged via the comparison colors. In another embodiment, the color-changing layer can be disposed in a connecting region (not shown) of the biochemical test chip, and when the biochemical test chip is inserted in a measuring instrument, the measuring instrument can read the color shown by the conjugated polymer in the color-changing layer via an optical system, and warn the user that the biochemical test chip is damaged via a method of display or sound. In an embodiment, the biochemical sensor is not limited to the biochemical test chip, and can also be, for instance, a biochemical cartridge.

In the present embodiment, the color-changing layer 160 can further include a monomer or a sensitizer. The monomer can be used to increase the color identification of the color-changing layer 160 and increase the stability of the conjugated polymer in the color-changing layer 160. In other words, in the present embodiment, the color-changing layer 160 having different colors can be formed by adding different amounts or different types of monomer, such that the identification of the changed color in the color-changing layer 160 is also different as a result. In an embodiment, the monomer can be, for instance, an amorphous polymer, chlorostyrene, ethylene, propene, butene, isoamylene, vinyl acetate, vinyl propionate, vinyl benzoate, vinyl butyrate, methyl acrylate, ethyl acrylate, butyl acrylate, 2-ethylhexyl acrylate, phenyl acrylate, methyl methacrylate, ethyl methacrylate, butyl methacrylate, vinyl methyl ether, ethoxyethene, butyl vinyl ether, methyl vinyl ketone, acetylacetone, polyvinyl, polypropylene, a polyester resin, a polyurethane resin, an epoxy resin, a silicone resin, a modified resin, paraffins, or a combination thereof.

In the present embodiment, to respond to the reaction of different reactive layers 140 to environmental conditions, a sensitizer can be added in the color-changing layer 160 to adjust the reactivity of cationic polymerization and/or radical polymerization of the conjugated polymer active energy. The sensitizer can be, for instance, an unsaturated ketone, a 1,2-diketone derivative, a benzo derivative, a fluorene derivative, a naphthoquinone derivative, an anthraquinone derivative, a xanthenes derivative, a coumarin derivative, a gallocyanine derivative, a merocyanine-based derivative, a polymethine derivative, an acridine derivative, a pyridazine derivative, an oxazine derivative, an indoline derivative, an azulene derivative, a porphyrins derivative, a tetraphenyl porphyrin derivative, a triarylmethane derivative, a phthalocyanin derivative, an annulene derivative, a spiropyrans derivative, a spirooxazine derivative, an organic ruthenium complex, or a combination thereof. The unsaturated ketone can be, for instance, a flavones derivative, dibenzalacetone, or a combination thereof, and the 1,2-diketone derivative can be, for instance, a benzyl derivative, a camphorquinone derivative, or a combination thereof

Moreover, in the present embodiment, an initiator can be added in the color-changing layer 160. The initiator can polymerize the conjugated polymer free radical to adjust the reaction rate constant of the color-changing layer 160. The initiator can be organic peroxide which generates the free radical via, for instance, a specific temperature condition, but the invention is not limited thereto. The initiator can be, for instance, isobutyl peroxide, α,α'-bis(neodecanoylperoxy) diisopropylbenzene, cumyl peroxyneodecanoate, bis-n-propyl peroxydicarbonate, bis-s-butyl peroxydicarbonate, 1,1,3,3-tetramethylbutyl neodecanoate, bis(4-t-butylcyclohexyl)peroxydicarbonate, 1-cyclohexyl-1-methyl ethyl peroxyneodecanoate, bis-2-ethoxy ethyl peroxydicarbonate, bis(ethylhexylperoxy)dicarbonate, t-hexyl neodecanoate, bismethoxy butyl peroxydicarbonate, bis(3-methyl-3-methoxybutylperoxy)dicarbonate, t-butyl peroxyneodecanoate, t-hexyl peroxypivalate, 3,5,5-trimethyl hexanoyl peroxide, octanoyl peroxide, lauroyl peroxide, stearoyl peroxide, 1,1,3,3-tetramethyl butyl peroxy-2-ethyl hexanoate, succinic peroxide, 2,5-dimethyl-2,5-bis(2-ethylhexanoyl)hexane, 1-cyclohexyl-1-methylethyl peroxy-2-ethylhexanoate, t-hexyl peroxy-2-ethyl hexanoate, 4-methylbenzoyl peroxide, t-butyl peroxy-2-ethylhexanoate, m-toluoyl benzoyl peroxide, benzoyl peroxide, t-butylperoxy isobutyrate, 1,1-bis(t-butylperoxy)-2-methyl-cyclohexane, 1,1-bis(t-butylperoxy)-3,3,5-trimethylcyclohexane, 1,1-bis(t-hexylperoxy)cyclohexane, 1,1-bis(t-butylperoxy)-3,3,5-trimethylcyclohexane, 1,1-bis(t-butylperoxy)cyclohexane, 2,2-bis(4,4-di-butylperoxycyclohexyl)propane, 1,1-bis(t-butylperoxy)cyclododecane, t-hexyl peroxy isopropyl monocarbonate, t-butylperoxy maleic acid, t-butyl peroxy-3,5,5-trimethylhexanoate, t-butylperoxy laurate, 2,5-dimethyl-2,5-bis(m-toluoylperoxy)hexane, t-butylperoxy isopropyl monocarbonate, t-butylperoxy-2-ethylhexyl monocarbonate, t-hexyl peroxybenzoate, 2,5-dimethyl-2,5-bis(benzoylperoxy)hexane, t-butylperoxyacetate, 2,2-bis(t-butylperoxy)butane, t-butylperoxybenzoate, n-butyl4,4-bis(t-butylperoxy)valerate, di-t-butylperoxyisophthalate, α,α'-bis(t-butylperoxy)diisopropylbenzene, dicumylperoxide, 2,5-dimethyl-2,5-di(t-butylperoxy)hexane, t-butylcumylperoxide, p-menthane hydroperoxide, 2,5-dimethyl-2,5-bis(t-butylperoxy)hexyne, diisopropylbenzene hydroperoxide, t-butyl trimethylsilyl peroxide, 1,1,3,3-tetramethyl butyl hydroperoxide, cumene hydroperoxide, t-hexyl hydroperoxide, t-butyl hydroperoxide, 2,3-dimethyl-2,3-diphenylbutane, or a combination thereof.

The invention does not limit the type or the quantity of the initiator. In another embodiment, the color-changing layer 160 can contain two or more than two initiators reacting to different temperatures, such that the color-changing layer 160 can change color in response to different environmental temperatures.

It should be mentioned that, the activity rate of change (such as activity decreasing rate of oxidoreductase) of the reactive layer 140 is equal to the concentration rate of change (such as concentration rate of change of conjugated polymer) of the color-changing layer 160. Therefore, when the activity decreasing rate of oxidoreductase in the reactive layer 140 is known (can be known from, for instance, an experiment), the concentration rate of change of the conjugated polymer in the color-changing layer 160 can be defined by formula (1) and formula (2) as shown below:

$$A = -\frac{\ln\left(\dfrac{C_i}{C_0}\right)}{K} \quad (1)$$

$$K = e^{\left(a + \frac{b}{T}\right)} \quad (2),$$

wherein,

K is a reaction rate constant of the color-changing layer 160 defined by formula (2);

T is a temperature in Kelvin;

a and b are experiment constants, wherein a is an intercept of the reaction rate constant K of the color-changing layer 160 and the temperature T, and b is a slope of the reaction rate constant K of the color-changing layer 160 and the temperature T;

$C_0$ is an original concentration of the color-changing layer 160;

$C_i$ is a concentration change to be shown by the color-changing layer 160, wherein the concentration change is defined as a concentration change when the reactive layer 140 loses the original measurement function;

A is a stability duration of the reactive layer 140 or the color-changing layer 160 at the temperature T.

It should be mentioned that, K is a reaction rate constant of the color-changing layer 160, and can be adjusted by the concentrations of the conjugated polymer, the monomer, the sensitizer, and the initiator. In other words, in the present embodiment, the color change of the color-changing layer 160 at a specific temperature and time can be controlled via the concentrations of the conjugated polymer, the monomer, the sensitizer, and the initiator.

However, the invention does not limit the color performance and the range of change. The only condition is that the color shown by the color-changing layer 160 of the present embodiment when the original measurement function is lost due to an environmental factor (such as ambient temperature) and the color shown at product completion (or when leaving the factory) are different by 1 or more color card code difference. In an embodiment, the color shown by the color-changing layer 160 when the original measurement function is lost due to an environmental factor (such as ambient temperature) and the color shown at product completion can show 7 or more color card code differences.

The "color card code" is the color code indicated in PANTONE MATCHING SYSTEM® published by Pantone Inc. In the case that the yellow of PANTONE MATCHING SYSTEM® color card code PMS 106C mixed from Pantone Yellow 8 pts, Pantone Warm Red 0.125 pts, and Pantone Trans. Wt. 56 pts is used, related description is provided in U.S. Patent Publication No. 5734800, titled "six-color process system", which is incorporated herein as a reference to the above. Regarding the color card code difference as the code difference in PANTONE MATCHING SYSTEM®, as an example, PMS 106C and PMS 105C or PMS 107C show one color card code difference.

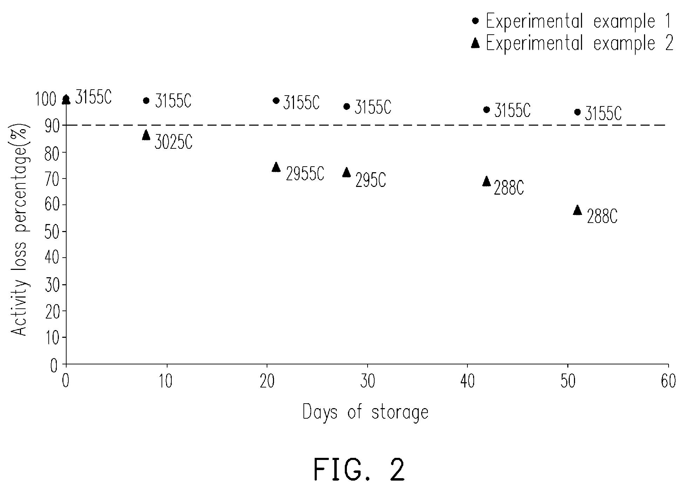

FIG. 2 is a graph of activity loss of the reactive layers of experimental examples 1 and 2.

<Experiments>

To prove the practicality of the invention, the following experimental examples are provided to more specifically describe the invention. Although the following experiments are described, the materials used and the amount and ratio thereof, as well as handling details and handling process...etc., can be suitably modified without exceeding the scope of the invention. Accordingly, restrictive interpretation should not be made to the invention based on the experiments described below.

(Experimental example 1)

A color-changing layer having a conjugated polymer was disposed on a biochemical test chip, wherein the biochemical test chip was used to measure cholesterol. Then, the biochemical test chip was placed in an environment having a temperature of 30 degrees Celsius continuously for 51 days, and the color change of the color-changing layer was observed. The color change of the color-changing layer is as shown in FIG. 2.

(Experimental example 2)

A color-changing layer having a conjugated polymer was disposed on a biochemical test chip, wherein the biochemical test chip was used to measure cholesterol. Then, the biochemical test chip was placed in an environment having a temperature of 50 degrees Celsius continuously for 51 days, and the color change of the color-changing layer was observed. The color change of the color-changing layer is as shown in FIG. 2.

The results of FIG. 2 show that, in comparison to experimental example 1, since the biochemical test chip of experimental example 2 is placed at a high temperature of 50 Celsius degrees, the oxidoreductase activity of experimental example 2 may be reduced, thus causing the oxidoreductase of experimental example 2 to lose the original activity or lose a portion or even all of the response capability. Moreover, the electron transfer regulator (such as potassium ferrocyanide) of experimental example 2 is also affected by light or heat, thus causing structural change, and the structural change is mostly an irreversible reaction. Therefore, the activity of the biochemical test chip of experimental example 2 is reduced from 100% (day 0) to less than 90% (day 8), which is outside the allowable range (i.e., 90%) of sensing cholesterol. In other words, after 8 days, the biochemical test chip of experimental example 2 permanently loses the original function of measuring cholesterol.

Moreover, as shown in FIG. 2, the color of the color-changing layer of experimental example 1 is the same (PMS 3155C) from day 0 to day 51. The color of the color-changing layer of experimental example 2 is changed from the color (PMS 3155C) of day 0 to the color (PMS 3025C) of day 8, and then changed to the color (PMS 288C) of day 51. Therefore, the user can compare the colors of the color-changing layer of the biochemical test chip via comparison colors pre-printed on a specimen jar to learn whether the biochemical test chip is damaged.

Based on the above, in the invention, via the color-changing layer disposed at least on a region outside the reactive layer and having a conjugated polymer, the color thereof can be changed with a change in an environmental factor. Moreover, the color change of the color-changing layer can be displayed as various texts, numbers, figures, patterns, or symbols. Therefore, the user can learn whether the biochemical test chip is damaged without having to place a sample in a reaction region. As a result, not only is wear and tear of the sample reduced, the risk of detection error of the biochemical test chip can also be reduced.

## Claims

1. A biochemical test chip (100), comprising:

   an insulating substrate (110);
   an electrode unit (120) located on the insulating substrate (110);
   a first insulating septum (130) located on the electrode unit (120) and having an opening (132), the opening (132) exposing a portion of the electrode unit (120);
   a reactive layer (140) located in the opening (132);
   a second insulating septum (150) located on the first insulating septum (130); and
   a color-changing layer (160) disposed at least on a region outside the reactive layer (140), wherein a color of the color-changing layer (160) is changed with a change in an environmental factor.

2. The biochemical test chip (100) of claim 1, wherein the color-changing layer (160) is disposed on a top or a bottom of the insulating substrate (110), a top or a bottom of the first insulating septum (130), or a top or a bottom of the second insulating septum (150).

3. The biochemical test chip (100) of claim 1, wherein a material of the color-changing layer (160) comprises a conjugated polymer.

4. The biochemical test chip (100) of claim 3, wherein the conjugated polymer comprises an aromatic hydrocarbon compound, a non-aromatic hydrocarbon compound, a trans-aromatic hydrocarbon compound, or a combination thereof.

5. The biochemical test chip (100) of claim 4, wherein the aromatic hydrocarbon compound comprises a polycyclic aromatic hydrocarbon compound, a phenyl compound, a crystal violet derivative, or a combination thereof,
   wherein the polycyclic aromatic hydrocarbon compound comprises a triarylmethane compound, a fluoran compound, a phenothiazine compound, a thiofluoran compound, a xanthene compound, a spiropyran compound, a chromenopyrazole compound, a methine compound, a rhodaminelactam compound, a quinazoline compound, a diazaxanthene compound, a bislactone compound, or a combination thereof,
   wherein the phenyl compound comprises a cresol red derivative, a thymol blue derivative, an aniline yellow derivative, a 2,4-dinitrophenol derivative, a bromophenol blue derivative, a methyl orange derivative, a bromcresol green derivative, a methyl red derivative, an eriochrome black T derivative, a bromcresol purple derivative, an alizarin derivative, an m-nitrophenol derivative, an o-cresolphthalein derivative, a phenolphthalein derivative, a thymolphthalein derivative, an alizarin yellow R derivative, an indigo carmine derivative, a malachite green derivative, a phenyl tribromomethyl sulfone derivative, a Victoria blue B derivative, a Victoria green G derivative, a phthalocyanine derivative, a phthaloyanine green G derivative, or a combination thereof.

6. The biochemical test chip (100) of claim 1, wherein the color-changing layer (160) further comprises a monomer, and the monomer comprises an amorphous polymer, chlorostyrene, ethylene, propene, butene, isoamylene, vinyl acetate, vinyl propionate, vinyl benzoate, vinyl butyrate, methyl acrylate, ethyl acrylate, butyl acrylate, 2-ethylhexyl acrylate, phenyl acrylate, methyl methacrylate, ethyl methacrylate, butyl methacrylate, vinyl methyl ether, ethoxyethene, butyl vinyl ether, methyl vinyl ketone, acetylacetone, polyvinyl, polypropylene, a polyester resin, a polyurethane resin, an epoxy resin, a silicone resin, a modified resin, paraffins, or a combination thereof.

7. The biochemical test chip (100) of claim 1, wherein the color-changing layer (160) further comprises a sensitizer, and the sensitizer comprises unsaturated ketone, a 1,2-diketone derivative, a benzo derivative, a fluorene derivative, a naphthoquinone derivative, an anthraquinone derivative, a xanthenes derivative, a coumarin derivative, a gallocyanine derivative, a merocyanine-based derivative, a polymethine derivative, an acridine derivative, a pyridazine derivative, an oxazine derivative, an indoline derivative, an azulene derivative, a porphyrins derivative, a tetraphenyl porphyrin derivative, a triarylmethane derivative, a phthalocyanin derivative, an annulene derivative, a spiropyrans derivative, a spirooxazine derivative, an organic ruthenium complex, or a combination thereof,
   wherein the unsaturated ketone comprises a flavones derivative, dibenzalacetone, or a combination thereof, and the 1,2-diketone derivative comprises a benzyl derivative, a camphorquinone derivative, or a combination thereof.

8. The biochemical test chip (100) of claim 1, wherein a forming method of the color-changing layer (160) comprises a coating method, an inkjet printing method, a mesh printing method, or a distributed printing method.

9. The biochemical test chip (100) of claim 1, wherein the environmental factor comprises light, temperature, humidity, oxygen content, or a combination thereof.

10. The biochemical test chip (100) of claim 1, wherein a color change of the color-changing layer (160) displays a text, a number, a figure, a pattern, a symbol, or a combination thereof.

11. The biochemical test chip (100) of claim 1, wherein the color-changing layer (160) further comprises an initiator, and the initiator comprises organic peroxide,

   wherein the organic peroxide comprises isobutyl peroxide, $\alpha,\alpha'$-bis(neodecanoylperoxy)diisopropylbenzene, cumyl peroxyneodecanoate, bis-n-propyl peroxydicarbonate, bis-s-butyl peroxydicarbonate, 1,1,3,3-tetramethylbutyl neodecanoate, bis(4-t-butylcyclohexyl)peroxydicarbonate, 1-cyclohexyl-1-methyl ethyl peroxyneodecanoate, bis-2-ethoxy ethyl peroxydicarbonate, bis(ethylhexylperoxy)dicarbonate, t-hexyl neodecanoate, bismethoxy butyl peroxydicarbonate, bis(3-methyl-3-methoxybutylperoxy)dicarbonate, t-butyl peroxyneodecanoate, t-hexyl peroxypivalate, 3,5,5-trimethyl hexanoyl peroxide, octanoyl peroxide, lauroyl peroxide, stearoyl peroxide, 1,1,3,3-tetramethyl butyl peroxy-2-ethyl hexanoate, succinic peroxide, 2,5-dimethyl-2,5-bis(2-ethylhexanoyl)hexane, 1-cyclohexyl-1-methylethyl peroxy-2-ethylhexanoate, t-hexyl peroxy-2-ethyl hexanoate, 4-methylbenzoyl peroxide, t-butyl peroxy-2-ethylhexanoate, m-toluoyl benzoyl peroxide, benzoyl peroxide, t-butylperoxy isobutyrate, 1,1-bis(t-butylperoxy)-2-methyl-cyclohexane, 1,1-bis(t-butylperoxy)-3,3,5-trimethylcyclohexane, 1,1-bis(t-hexylperoxy)cyclohexane, 1,1-bis(t-butylperoxy)-3,3,5-trimethylcyclohexane, 1,1-bis(t-butylperoxy)cyclohexane, 2,2-bis(4,4-di-butylperoxycyclohexyl)propane, 1,1-bis(t-butylperoxy)cyclododecane, t-hexyl peroxy isopropyl monocarbonate, t-butylperoxy maleic acid, t-butyl peroxy-3,5,5-trimethylhexanoate, t-butylperoxy laurate, 2,5-dimethyl-2,5-bis(m-toluoylperoxy)hexane, t-butylperoxy isopropyl monocarbonate, t-butylperoxy-2-ethylhexyl monocarbonate, t-hexyl peroxybenzoate, 2,5-dimethyl-2,5-bis(benzoylperoxy)hexane, t-butylperoxyacetate, 2,2-bis(t-butylperoxy)butane, t-butylperoxybenzoate, n-butyl-4,4-bis(t-butylperoxy)valerate, di-t-butylperoxyisophthalate, $\alpha,\alpha'$-bis(t-butylperoxy)diisopropylbenzene, dicumylperoxide, 2,5-dimethyl-2,5-di(t-butylperoxy)hexane, t-butylcumylperoxide, p-menthane hydroperoxide, 2,5-dimethyl-2,5-bis(t-butylperoxy)hexyne, diisopropylbenzene hydroperoxide, t-butyl trimethylsilyl peroxide, 1,1,3,3-tetramethyl butyl hydroperoxide, cumene hydroperoxide, t-hexyl hydroperoxide, t-butyl hydroperoxide, 2,3-dimethyl-2,3-diphenylbutane, or a combination thereof.

12. The biochemical test chip (100) of claim 1, wherein an activity rate of change of the reactive layer (140) is equal to a concentration rate of change of the color-changing layer (160).

13. The biochemical test chip (100) of claim 12, wherein the concentration rate of change of the color-changing layer (160) is defined by formula (1) and formula (2) as shown below:

$$A = -\frac{\ln\left(\dfrac{C_i}{C_0}\right)}{K} \tag{1}$$

$$K = e^{(a+\frac{b}{T})} \tag{2},$$

wherein,

   K is a reaction rate constant of the color-changing layer (160) defined by formula (2);
   T is a temperature in Kelvin;
   a and b are experiment constants, wherein a is an intercept of the reaction rate constant of the color-changing layer (160) and the temperature, and b is a slope of the reaction rate constant of the color-changing layer (160) and the temperature;
   $C_0$ is an original concentration of the color-changing layer (160);
   $C_i$ is a concentration change to be shown by the color-changing layer (160), wherein the concentration change is defined as a concentration change when the reactive layer (140) loses the original measurement function;
   A is a stability duration of the reactive layer (140) or the color-changing layer (160) at the temperature T.

14. The biochemical test chip (100) of claim 13, wherein the reaction rate constant of the color-changing layer (160) at a specific temperature is adjusted by a concentration of a compound in the color-changing layer (160).

15. The biochemical test chip (100) of claim 14, wherein the compound in the color-changing layer (160) comprises a conjugated polymer, a monomer, a sensitizer, an initiator, or a combination thereof.

FIG. 1

FIG. 2

Europäisches
Patentamt

European
Patent Office

Office européen
des brevets

# EUROPEAN SEARCH REPORT

Application Number

EP 16 18 2537

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/108968 A1 (MATSUSHITA ELECTRIC IND CO LTD [JP]; MIYASHITA MARIKO; TANIIKE YUKO; Y) 17 November 2005 (2005-11-17) * the whole document * * abstract * * figure 1 * * claims 1-11 * | 1-5,7-15 | INV. G01N33/52 G01N21/78 G01N33/543 G01N31/22 |
| X | US 2010/159610 A1 (SUN HOI-CHEONG STEVE [US] ET AL) 24 June 2010 (2010-06-24) * the whole document * * abstract * * paragraph [0042] * * paragraph [0060] - paragraph [0063] * * paragraph [0065] * * figures 1-2 * | 1-6 | |
| A | US 2012/262298 A1 (BOHM SEBASTIAN [US] ET AL) 18 October 2012 (2012-10-18) * the whole document * * abstract * * paragraph [0321] * * paragraph [0269] * | 1-15 | TECHNICAL FIELDS SEARCHED (IPC) G01N |
| A | WO 2014/063419 A1 (GUANGZHOU WONDFO BIOTECH CO LTD [CN]) 1 May 2014 (2014-05-01) * the whole document * * abstract * * figures 1-2 * | 1-15 | |

The present search report has been drawn up for all claims

| Place of search | Date of completion of the search | Examiner |
|---|---|---|
| The Hague | 24 February 2017 | Gall-Truchot, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 16 18 2537

This annex lists the patent family members relating to the patent documents cited in the above-mentioned European search report.
The members are as contained in the European Patent Office EDP file on
The European Patent Office is in no way liable for these particulars which are merely given for the purpose of information.

24-02-2017

| Patent document cited in search report | | Publication date | Patent family member(s) | | Publication date |
|---|---|---|---|---|---|
| WO 2005108968 | A1 | 17-11-2005 | NONE | | |
| US 2010159610 | A1 | 24-06-2010 | CA | 2740878 A1 | 24-06-2010 |
| | | | CN | 102227627 A | 26-10-2011 |
| | | | CN | 104297248 A | 21-01-2015 |
| | | | DK | 2380009 T3 | 04-05-2015 |
| | | | EP | 2380009 A1 | 26-10-2011 |
| | | | EP | 2851676 A2 | 25-03-2015 |
| | | | ES | 2535289 T3 | 08-05-2015 |
| | | | HK | 1202925 A1 | 09-10-2015 |
| | | | US | 2010159610 A1 | 24-06-2010 |
| | | | US | 2014295571 A1 | 02-10-2014 |
| | | | US | 2016003749 A1 | 07-01-2016 |
| | | | WO | 2010071708 A1 | 24-06-2010 |
| US 2012262298 | A1 | 18-10-2012 | EP | 2697650 A2 | 19-02-2014 |
| | | | JP | 2014514093 A | 19-06-2014 |
| | | | US | 2012262298 A1 | 18-10-2012 |
| | | | US | 2012265035 A1 | 18-10-2012 |
| | | | US | 2012265036 A1 | 18-10-2012 |
| | | | US | 2012265037 A1 | 18-10-2012 |
| | | | US | 2014114153 A1 | 24-04-2014 |
| | | | US | 2014114156 A1 | 24-04-2014 |
| | | | US | 2016018246 A1 | 21-01-2016 |
| | | | US | 2016073941 A1 | 17-03-2016 |
| | | | US | 2016157758 A1 | 09-06-2016 |
| | | | US | 2016198986 A1 | 14-07-2016 |
| | | | WO | 2012142502 A2 | 18-10-2012 |
| WO 2014063419 | A1 | 01-05-2014 | CN | 102901808 A | 30-01-2013 |
| | | | WO | 2014063419 A1 | 01-05-2014 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5734800 A